# DEMANDE DE BREVET EUROPEEN

(11) **EP 0 647 639 A1**
(43) Date de publication de la demande: **12.04.1995**
(21) Numéro de dépôt: 94402262.3
(22) Date de dépôt: 10.10.1994
(51) Int. Cl.: C07D 401/04, A61K 31/445

(54) **Hétéroarylpipéridines, procédé pour leur préparation et compositions pharmaceutiques les contenant**

(30) Priorité: 11.10.1993 EP 93402498
(71) Demandeur: SANOFI, F-75008 Paris (FR); MIDY S.p.A., I-20137 Milano (IT)
(72) Inventeur: Baroni, Marco, I-20010 Vanzago, Milano (IT); Croci, Tiziano, I-20155 Milano (IT); Landi, Marco, Bussero, Milano (IT); Guzzi, Umberto, I-20100 Milano (IT); Nisato, Dino, F-34680 Saint Georges D'Orques (FR)
(74) Mandataire: Le Roux, Martine

(57) **Abrégé**

La présente invention concerne de nouvelles hétéroarylpipéridines de formule (I) 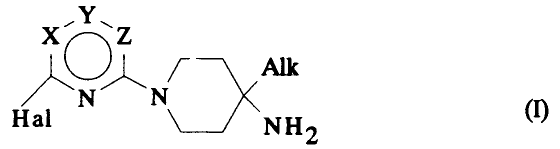
dans laquelle Hal représente un atome d'halogène, Alk représente un groupe (C₁-C₄)alkyle, X, Y et Z représentent chacun -CH= ou l'un d'entre eux est un atome d'azote et les autres représentent -CH=, ainsi que leurs sels, un procédé pour leur préparation et leur utilisation en tant que médicaments à action agoniste 5-HT₃.

## Description

La présente invention concerne de nouvelles 1-hétéroaryl-4-alkyl-4-aminopipéridines à activité agoniste sérotoninergique 5HT₃, le procédé pour leur préparation, ainsi que les compositions pharmaceutiques les contenant.

Certaines 4-amino-1-(2-pyridinyl)pipéridines sont décrites dans le brevet EP-B-21973, en tant qu'agents anorexigènes. L'activité sérotoninergique 5HT₃ agoniste de ces composés est illustrée dans la demande EP-A-506 545.

On a maintenant trouvé que l'introduction d'un groupe alkyle en position 4 de ces 4-amino-1-(2-pyridinyl)pipéridines conduit à des produits agonistes 5HT₃ plus puissants et plus sélectifs que les produits connus et on a aussi trouvé que les dérivés ayant un noyau pyrimidinique ou pyrazinique au lieu de la pyridine ont la même activité et une très bonne sélectivité.

Ainsi, selon un de ses aspects, la présente invention a pour objet de nouvelles 1-hétéroaryl-4-alkyl-4-aminopipéridines de formule (I) : 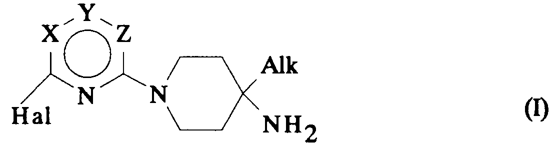
dans laquelle Hal représente un atome d'halogène, Alk représente un groupe (C₁-C₄) alkyle, X, Y et Z représentent chacun -CH= ou l'un d'entre eux est un atome d'azote et les deux autres représentent -CH=, ainsi que leurs sels, pharmaceutiquement acceptables ou non.

Dans la présente description, les termes "atome d'halogène" et "halo" désignent de préférence le chlore et le brome mais peuvent aussi représenter un atome de fluor ou d'iode; le terme "(C₁-C₄)alkyle" désigne un radical alkyle linéaire ou ramifié contenant 1 à 4 atomes de carbone, tels que les radicaux méthyle et éthyle.

Les composés de formule (I) dans laquelle Hal représente un atome de chlore et Alk représente un groupe méthyle et leurs sels sont des composés particulièrement avantageux.

Parmi ces composés,
- la 4-amino-1-(6-chloro-2-pyridinyl)-4-méthylpipéridine et ses sels pharmaceutiquement acceptables, notamment son chlorhydrate;
- la 4-amino-1-(4-chloro-2-pyrimidinyl)-4-méthylpipéridine et ses sels pharmaceutiquement acceptables, notamment son maléate,
- la 4-amino-1-(2-chloro-4-pyrimidinyl)-4-méthylpipéridine et ses sels pharmaceutiquement acceptables, notamment son maléate, et
- la 4-amino-1-(6-chloro-2-pyrazinyl)-4-méthylpipéridine et ses sels pharmaceutiquement acceptables, notamment son chlorhydrate et son maléate,

sont particulièrement préférées.

Selon un autre de ses aspects, la présente invention concerne un procédé pour la préparation des 1-hétéroaryl-4-alkyl-4-aminopipéridines de formule (I) ci-dessus caractérisé en ce que:
(a) on traite une 4-alkyl-1,2,3,6-tétrahydropyridine NH-protégée de formule (II) 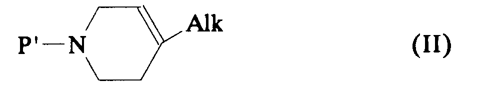 dans laquelle Alk représente un groupe alkyle de 1 à 4 atomes de carbone et P' est un groupe NH-protecteur clivable par réduction, avec acétonitrile et acide sulfurique et on isole une 4-acétylamino-4-alkylpipéridine NH-protégée de formule (III') 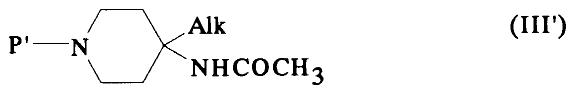 dans laquelle P' et Alk sont tels que définis ci-dessus, puis, éventuellement,
   (a') on soumet le composé (III') ainsi obtenu à une NH₂-déprotection par hydrolyse pour obtenir une 4-alkyl-4-aminopipéridine NH-protégée de formule (IV) 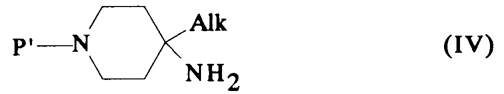 dans laquelle P' et Alk sont tels que définis ci-dessus; et
   (a'') on protège le groupe amino primaire par un groupe, autre que le groupe acétyle, clivable par hydrolyse;
(b) on soumet la 4-alkyl-4-aminopipéridine diprotégée ainsi obtenue de formule (III) 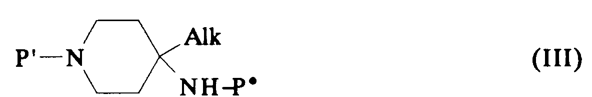 dans laquelle P' et Alk sont tels que définis ci-dessus et P^{·} représente un groupe NH₂-protecteur clivable par hydrolyse, à une NH-déprotection par réduction;
(c) on traite la 4-alkyl-4-aminopipéridine NH₂-protégée ainsi obtenue de formule (V) 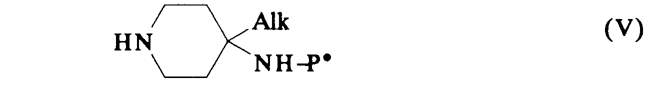 dans laquelle P^{·} et Alk sont tels que définis ci-dessus, avec un dihalohéteroaryle de formule (VI) 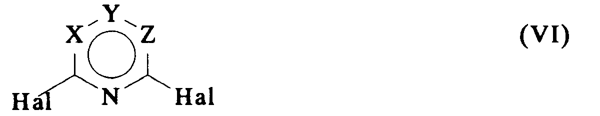 dans laquelle Hal, X, Y et Z sont tels que définis ci-dessus ;
d) on hydrolyse le composé ainsi obtenu de formule (VII) 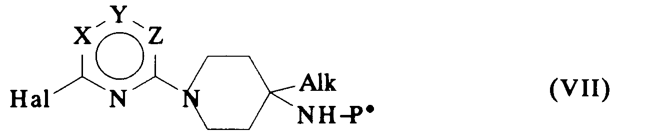 dans laquelle Hal est un halogène et X, Y, Z, Alk et P^{·} sont tels que définis ci-dessus; et
(e) on isole le produit (I) ainsi obtenu tel quel ou sous forme d'un de ses sels ou on le transforme en un de ses sels.

Les termes "NH-protection", "NH-déprotection", "NH-protégé", "NH-déprotégé", tels qu'utilisés dans la présente description et dans les revendications et se rapportant au groupe "NH-déprotecteur" P', se réfèrent à la protection et à la déprotection de l'amine secondaire, à savoir de l'azote constituant la position 1 de la pipéridine. De façon analogue, les termes "NH₂-protection", "NH₂-déprotection", "NH₂-protégé", "NH₂-déprotégé", se rapportant au groupe "NH₂-protecteur" P^{·}, se réfèrent à la protection et à la déprotection de l'amine primaire, à savoir de l'amine dans la position 4 de la pipéridine.

Dans la présente description, le terme "groupe protecteur" désigne un groupe protecteur du groupe amino du type de ceux qu'on emploie dans la synthèse des peptides. Par exemple, les groupes acyles, tels que le formyle, l'acétyle, le propionyle le phénylacétyle, le phénoxyacétyle et similaires; un groupe alcoxycarbonyle tel que le t-butoxycarbonyle (BOC) et similaires; un groupe alcoxyalkylcarbonyle tel que le méthoxypropionyle et similaires; un groupe alcoxycarbonyle substitué, tel que le trichloroéthoxycarbonyle et similaires; un groupe alkylcarbonyle substitué tel que le monochlorométhylcarbonyle, monochloroéthylcarbonyle, dichlorométhylecarbonyle, trichlorométhylcarbonyle, trichloroéthylcarbonyle, trichloropropylcarbonyle, trifluorométhylcarbonyle et similaires; un groupe arylalkyloxycarbonyle, tel que le benzyloxycarbonyle et similaires; un groupe arylalkyloxycarbonyle substitué, tel que le 4-nitrobenzyloxycarbonyle et similaires;un groupe benzyle; un groupe benzyle substitué; un groupe diphénylméthyle éventuellement substitué; un groupe trityle éventuellement substitué, tel que le 4-méthoxyphényldiphénylméthyle ou le di-(4-méthoxyphényl)phénylméthyle; un groupe silylant tel que le triméthylsilyle ou l'éthyldiméthylsilyle, ou le t-butyldiméthylsilyle et similaires.

Lesdits groupes protecteurs peuvent être clivés selon les méthodes conventionnelles par exemple par réduction ou hydrolyse. Une description plus détaillée de ces groupes amino-protecteurs ainsi que des méthodes pour leur préparation et leur élimination est donnée par exemple par T.W. Greene, "Protective Groups in Organic Synthesis", John Wiley & Sons, 1981 et par J.F.W. McOmie, "Protective Groups in Organic Chemistry", Plenum Press, 1973.

Dans la présente description, le groupe NH-protecteur représenté par P' est par définition un groupe clivable par réduction et le groupe NH₂-protecteur représenté par P^{·} est par définition un groupe clivable par hydrolyse.

Un groupe NH-protecteur P' préférentiel est le groupe benzyle, éventuellement substitué sur le cycle aromatique, par exemple par un groupe méthoxy ou nitro ou par un atome de chlore, de préférence en position 4, le groupe benzyle non-substitué étant particulièrement préféré. Ce groupe est éliminé par hydrogénation catalytique, en utilisant de préférence du palladium sur charbon comme catalyseur.

Des groupes NH₂-protecteurs P^{·} préférentiels sont les groupes alcanoyle en C₁-C₄, trifluoroacétyle et alcoxycarbonyle en C₁-C₄, les groupes acétyle et t-butoxycarbonyle (BOC) étant particulièrement préférés. Ces groupes sont éliminés par hydrolyse en milieu acide, par exemple avec de l'acide chlorhydrique ou trifluoroacétique.

Les 1,2,3,6-tétrahydropyridines de départ de formule (II) où P' représente un groupe benzyle ou benzyle substitué, sont des produits connus en littérature. Ils peuvent être préparés selon les méthodes décrites dans le brevet DE 2101997, dans J. Am. Chem. Soc., 1985, 107, 1768-1769 et dans Organic Synthesis, 70, 111-119, 1992. Les composés de formule (II) où P' est un groupe protecteur clivable par réduction autre que le groupe benzyle, peuvent être préparés par la même méthode.

L'étape (a), qui est une réaction de Ritter, est conduite en utilisant un excès d'acétonitrile environ double par rapport au composé (II) de départ et en ajoutant au mélange composé (II)/acétonitrile un excès d'acide sulfurique concentré. Après chauffage entre 50° C et la température de reflux, de préférence à 60-80° C, pendant 18-30 heures, le composé de formule (III) est isolé par neutralisation de l'excès d'acide, extraction dans un solvant tel que l'éther ou le tétrahydrofurane, évaporation du solvant et reprise ou cristallisation du résidu.

Le composé (III') ainsi obtenu peut être directement soumis à l'étape (b). Pour des raisons de réactivité, il peut être cependant convenable de transformer le groupe acétyle en un autre groupe protecteur également clivable par hydrolyse. Ainsi, le composé (III'), dans l'étape supplémentaire éventuelle (a'), est soumis à une hydrolyse, avantageusement en milieu acide, et transformé en la 4-alkyl-4-aminopipéridine NH-protégée de formule (IV).

Cette NH₂-déprotection par clivage du groupe acétyle est effectuée avec un acide fort, comme l'acide chlorhydrique, par chauffage pendant 24-48 heures. Le produit de formule (IV) est isolé par neutralisation du mélange réactionnel et purification du résidu par cristallisation ou par chromatographie.

Ensuite, dans la deuxième étape supplémentaire éventuelle (a''), le composé (IV) est soumis à une NH₂-protection selon les méthodes classiques, illustrées par exemple dans les deux ouvrages cités ci-dessus, de façon à protéger l'amine primaire par un groupe protecteur clivable par hydrolyse autre que le groupe acétyle.

Cette NH₂-protection est effectuée en utilisant les réactifs appropriés connus sur la base du groupe NH₂-protecteur autre que l'acétyle choisi. Ainsi, si le groupe choisi est un groupe acyle tel que t-butoxycarbonyle, trifluoroacétyle, le composé (IV) est traité avec un dérivé fonctionnel dudit acide, par exemple le chlorure, l'anhydride, une anhydride mixte, un ester, un ester activé, par exemple l'ester de 4-nitrobenzyle. Si le groupe NH₂-protecteur choisi est un groupe non acylant, par exemple un groupe trityle ou triméthylsilyle, le composé (IV) est traité avec un halogénure du groupe choisi, par exemple le triphénylchlorométhane ou le triméthylchlorosilane ou avec un autre composé réactif bien connu comme le bis-triméthylsilylacétamide.

Dans l'étape (b) du procédé de la présente invention, la 4-amino-4-alkylpipéridine déprotégée de formule (III) obtenue à la fin de l'étape (a) ou, éventuellement, des étapes supplémentaires (a') et (a''), est soumise à une NH-déprotection sélective par élimination du groupe P'. Ladite NH-déprotection est effectuée par réduction, notamment par hydrogénation en présence d'un catalyseur approprié, le palladium sur charbon à 5-15 %, de préférence à 10 %, étant utilisable de façon générale. L'hydrogénation est effectuée dans un solvant organique polaire ou apolaire, protique ou aprotique par exemple dans un alcanol comme l'éthanol. Le composé NH-déprotégé de formule (V) est isolé selon les techniques conventionnelles, par exemple par filtration du catalyseur et de son support, évaporation du solvant et reprise du résidu par un solvant approprié, comme l'acétate d'éthyle ou un éther.

Dans l'étape (c), la réaction de condensation qui conduit au composé de formule (VII) est convenablement effectuée dans un solvant organique tel qu'un alcool, du diméthylformamide, du diméthylsulfoxyde, du sulfolane, de l'acétonitrile, de la pyridine et similaires, à une température généralement comprise entre 50°C et la température de reflux du solvant choisi, en présence d'un agent de condensation basique, tel qu'un hydroxyde, un carbonate ou un bicarbonate alcalin ou une amine tertiaire.

Le composé de formule (VII) est isolé selon les techniques habituelles, par exemple par évaporation du solvant, purification éventuelle par reprise du résidu avec un solvant approprié et chromatographie ou cristallisation.

Dans l'étape (d), on procède à la NH₂-déprotection par élimination du groupe P^{·} qui est hydrolysé notamment en milieu acide. Les conditions sont exactement les mêmes que celles illustrées précédemment pour l'étape (a'') si le groupe P^{·} est un groupe acétyle (formule III'). Pour d'autres groupes protecteurs, on utilise les techniques appropriées bien connues pour ce type de clivage. Par exemple, le groupe NH₂-protecteur préféré, le t-butoxycarbonyle, est clivé par action de l'acide trifluoroacétique.

Le produit de formule (I) ainsi obtenu est isolé selon les méthodes connues. Si la NH₂-déprotection de l'étape (d) du procédé de la présente invention est effectuée par hydrolyse en milieu acide, le produit de formule (I) peut être isolé comme sel d'addition avec l'acide utilisé. On peut également soumettre le mélange réactionnel obtenu après l'hydrolyse à une neutralisation avec une base telle que l'hydroxyde de sodium ou de potassium ou l'hydroxyde d'ammonium de façon à isoler la base libre de formule (I). Cette dernière peut, à son tour, être transformée dans un sel d'addition avec un acide autre que celui utilisé pour l'hydrolyse. Cette opération est souhaitable lorsque l'hydrolyse est effectuée avec un acide non pharmaceutiquement acceptable comme l'acide trifluoroacétique.

Les sels des composés de formule (I) sont aisément préparés de façon connue en soi par action de l'acide choisi sur le composé de formule (I) dissous, par exemple dans de l'acétone, de l'éther, ou dans un alcool tel que le méthanol, l'éthanol ou l'isopropanol.

Lorsque les sels des composés de formule (I) sont préparés pour être administrés en tant que médicaments, il faut que les acides employés soient des acides pharmaceutiquement acceptables; si on prépare des sels des composés de formule (I) dans un autre but, par exemple pour mieux purifier le produit ou pour mieux effectuer des essais analytiques, on peut alors utiliser n'importe quel acide d'addition.

Les sels pharmaceutiquement acceptables des composés de formule (I) comprennent les sels d'addition avec les acides minéraux ou organiques physiologiquement compatibles; par exemple les sels formés avec les acides chlorhydrique, bromhydrique, iodhydrique, nitrique, sulfurique, phosphorique, propionique, acétique, formique, benzoïque, maléique, fumarique, succinique, tartrique, citrique, aspartique, ascorbique, les acides sulfoniques, tels que l'acide méthanesulfonique, éthanesulfonique, 2-hydroxyéthanesulfonique, 2-naphtalènesulfonique, benzenesulfonique, p-toluènesulfonique.

Les composés nécessaires pour la synthèse des composés de formule (I), dont la préparation est illustrée ci-dessus, répondant aux formules (III), (IV), (V), (VII), sont nouveaux. Ils représentent un aspect ultérieur de la présente invention et peuvent être regroupés dans la formule (VIII) suivante : 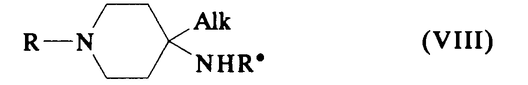
dans laquelle Alk représente un groupe (C₁-C₄) alkyle, R^{·} représente l'hydrogène ou un groupe protecteur P^{·}, tel que défini ci-dessus et R représente l'hydrogène, un groupe protecteur P', tel que défini ci-dessus, ou un hétérocycle de structure (IX) : 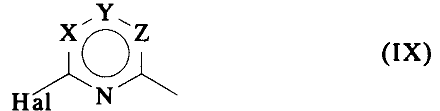
dans laquelle Hal, X, Y et Z sont définis comme précédemment, R^{·} étant autre que l'hydrogène lorsque R représente l'hydrogène ou un groupe de structure (IX).

Les sels éventuels des composés de formule (VIII) font également partie de l'invention.

Parmi les composés de formule (VIII) et leurs sels,
- la 1-benzyl-4-acétylamino-4-méthylpipéridine;
- la 1-benzyl-4-t-butoxycarbonylamino-4-méthylpipéridine;
- la 4-amino-1-benzyl-4-méthylpipéridine et ses sels;
- la 4-acetylamino-4-méthylpipéridine et ses sels;
- la 4-t-butoxycarbonylamino-4-méthylpipéridine et ses sels;
- la 4-acétylamino-1-(6-chloro-2-pyridinyl)-4-méthylpipéridine;
- la 4-t-butoxycarbonylamino-1-(6-chloro-2-pyridinyl)-4-méthylpipéridine;
- la 4-acétylamino-1-(4-chloro-2-pyrimidinyl)-4-méthylpipéridine;
- la 4-t-butoxycarbonylamino-1-(4-chloro-2-pyrimidinyl)-4-méthylpipéridine;
- la 4-acétylamino-1-(2-chloro-4-pyrimidinyl)-4-méthylpipéridine;
- la 4-t-butoxycarbonylamino-1-(2-chloro-4-pyrimidinyl)-4-méthylpipéridine;
- la 4-acétylamino-1-(6-chloro-2-pyrazinyl)-4-méthylpipéridine; et
- la 4-t-butoxycarbonylamino-1-(6-chloro-2-pyrazinyl)-4-méthylpipéridine
   sont particulièrement préférés.

Les composés de formule (I) tels que définis ci-dessus, ainsi que leurs sels pharmaceutiquement acceptables, présentent des propriétés pharmacologiques très intéressantes et inattendues. Notamment, ces produits possèdent une activité agoniste puissante et sélective pour le récepteur 5HT₃ de la sérotonine.

L'affinité des composés de formule (I) pour les récepteurs 5-HT₃ a été mise en évidence à l'aide d'essais de binding in vitro en utilisant les sites de liaison 5-HT₃ présents dans le cortex cérébral du rat (GJ. Kilpatrick, BJ. Jones et M.B. Tyers. Identification and distribution of 5-HT₃ receptors in rat brain using radioligand binding. Nature, 1987; **330**: 746-8) et comme ligand marqué le [³H] BRL 43694 (granisetron), un antagoniste des récepteurs 5-HT₃ puissant et spécifique.

La préparation des membranes et le test de binding ont été effectués selon la méthode décrite par Nelson et Thomas (D.R. Nelson et D.R. Thomas. [³H] BRL 43694 (granisetron), a specific ligand for 5HT₃ binding sites in rat brain cortical membranes, Biochem. Pharmacol., 1989; **38**: 1693-5).

Les résultats ont été évalués avec des méthodes d'ajustement ("fitting") non linéaire "Accufit saturation", pour les études de saturation (H.A.Feldman. Mathematical theory of complex ligand-binding systems at equilibrium: some methods of parameter fitting. Analyt. Biochem., 1972; **48**: 317-38) et "Accufit competition", pour les études de déplacement (H.A. Feldman, D. Rodbard et D. Levine. Mathematical theory of cross reactive radioimmunoassay and ligand-binding systems at equilibria. Analyt. Biochem., 1972; **45**: 530-56).

Dans ces essais in vitro les composés de formule (I) sont très puissants dans le déplacement du [³H] BRL 43694, utilisé à une concentration de 0,5 nM. Notamment, ils sont beaucoup plus puissants que la sérotonine et la 2-méthylsérotonine et aussi plus puissants et/ou plus sélectifs que les composés non alkylés à la position 4 de la pipéridine décrits dans la demande de brevet EP-A-506545.

De plus, les composés de formule (I) sont dépourvus d'activité sur les récepteurs autres que le 5HT₃, plus particulièrement sur les récepteurs sérotoninergiques, dopaminergiques, adrénergiques, histaminergiques et muscarinique, comme il ressort des tests de binding conventionnels.

Les composés de formule (I) possèdent aussi une activité procinétique intestinale liée à leur action 5HT₃ agoniste, selon le test d'élimination fécale chez le rat décrit dans la demande EP-A-506545 dans lequel, à doses faibles, ils ont montré une bonne activité de stimulation de l'élimination fécale.

Les composés de formule (I) sont peu toxiques; leur toxicité est compatible avec leur utilisation en tant que médicaments.

Les composés de formule (I) ainsi que leurs sels pharmaceutiquement acceptables peuvent donc être utilisés pour la préparation de médicaments indiqués dans le traitement et/ou la prophylaxie des altérations et des troubles du système sérotoninergique, périphérique et/ou central, là où les récepteurs 5HT₃ sont impliqués. Cette utilisation constitue un aspect ultérieur de la présente invention.

Ces médicaments peuvent être utilisés dans le traitement et/ou la prophylaxie des troubles dysthymiques, de la dépression, des troubles psychotiques ou dans les cas d'anxiété. Ils peuvent également être utilisés dans les désordres de la motricité intestinale, notamment dans le traitement de la constipation, du syndrome du côlon irritable (IBS, de l'anglais "Irritable Bowel Syndrome").

Ainsi, la présente invention concerne, selon un autre de ses aspects, des compositions pharmaceutiques renfermant, en tant que principe actif, un composé de formule (I) ou un de ses sels pharmaceutiquement acceptables, seul ou en association avec toute autre substance pharmacologiquement compatible, notamment destinées à l'administration par voie orale et/ou parentérale (sublinguale, rectale, transdermique).

Les compositions pharmaceutiques sus-mentionnées sont préparées selon les méthodes usuelles bien connues dans le domaine de la galénique, en mélangeant le principe actif de formule (I) ou un de ses sels pharmaceutiquement acceptables avec les excipients habituellement employés tels que le talc, la gomme arabique, le lactose, l'amidon, le stéarate de magnésium, les véhicules aqueux ou non aqueux, les corps gras d'origine animale ou végétale, les dérivés de la paraffine, les glycols, les agents mouillants, dispersants, émulsifiants, conservateurs, aromatisants, stabilisants, etc...

Pour l'administration par voie orale, des formes pharmaceutiquement appropriées comprennent les comprimés, les comprimés-retard, les dragées, les gélules, les suspensions, les solutions, les formes gastro-résistants, les formes liposomiques.

Pour l'administration parentérale, on peut préparer des compositions stériles injectables aqueuses ou non aqueuses, des suppositoires ou des microclystères pour l'administration rectale, des patches pour l'administration transdermique, ainsi que des formes pharmaceutiques aptes à l'administration oculaire ou nasale.

Le dosage approprié du principe actif doit être évalué selon la voie d'administration, les caractéristiques du sujet à traiter, tels que l'âge, le poids du corps ainsi que la gravité des affections à traiter.

Généralement, le dosage est compris entre 0,05 et 100 mg par jour notamment entre 0,1 et 50 mg par jour, plus convenablement entre 0,5 et 20 mg par jour, de préférence entre 1 et 10 mg par jour.

Ce dosage peut être subdivisé en doses unitaires à administrer plusieurs fois par jour, de préfèrence une à trois fois par jour. Chaque dose unitaire contient de 0,05 à 100 mg, avantageusement de 0,5 à 25 mg, de préférence de 1 à 10 mg de principe actif.

Les exemples qui suivent illustrent mieux l'invention sans toutefois la limiter.

### EXEMPLE 1

### Chlorhydrate de la 4-amino-1-(6-chloro-2-pyridinyl)-4-méthylpipéridine.

**a) 1-benzyl-4-acétylamino-4-méthylpipéridine - formule (VIII), Alk = méthyle, R**^{**·**}**= acétyle, R=benzyle**
On mélange 2,17 g (0,012 mole) de 1-benzyl-4-méthyl-1,2,3,6-tétrahydropyridine et 0,9 g (0,022 mole) d'acétonitrile, on y ajoute prudemment 3,6 ml d'acide sulfurique concentré et on chauffe à la température de 70°C pendant 24 heures. On verse le mélange encore chaud dans de l'eau/glace et on y ajoute une solution de carbonate de sodium à 20 % jusqu'à pH basique ; on extrait à l'éther éthylique, on sèche sur Na₂SO₄ et on évapore à sec sous pression réduite. On obtient ainsi 1,17 g du composé indiqué dans le titre (solide blanc, p.f. 103-105°C).
**b) 4-acétylamino-4-méthylpipéridine - formule (VIII), Alk = méthyle, R**^{**·**}**= acétyle, R = H.**
On hydrogène un mélange de 0,7 g (0,00284 mole) de 1-benzyl-4-acétylamino-4-méthylpipéridine obtenue selon l'étape (a) et 0,07 g de Pd/C à 10 % dans 10 ml d'alcool éthylique à la pression ambiante. Après 6 heures environ, on filtre le catalyseur, on évapore à sec et on obtient une huile blanche qui est traitée avec 2 ml d'éther éthylique. Ainsi on isole 0,4 g du composé du titre (solide blanc, p.f. 75-78°C).
**c) 4-acétylamino-1-(6-chloro-2-pyridinyl)-4-méthylpipéridine - formule (VIII) Alk=méthyle, R**^{**·**}**= acétyle, R = structure (IX) où Hal = Cl, X=Y=Z=CH.**
On chauffe au reflux un mélange de 9 g (0,063 mole) de 2,6 - dichloropyridine, 8 g (0,051 mole) de 4-acétylamino-4-méthylpipéridine obtenue selon l'étape (b) et 9,1 g (0,066 mole) de K₂CO₃ dans 75 ml de n-pentanol pendant 60 heures. On évapore à sec, on reprend le résidu à l'eau (80 ml environ) et on extrait à l'éther éthylique; on sèche la phase organique sur Na₂SO₄ et on l'évapore à sec. On obtient un solide qui, traité avec de l'hexane, donne 6,9 g du composé indiqué ci-dessus (p.f. 77-80° C).
**d) Chlorhydrate de 4-amino-1-(6-chloro-2-pyridinyl)-4-méthylpipéridine.**
On chauffe au reflux pendant 24 heures un mélange de 6,8 g (0,025 mole) de 4-acétylamino-1-(6-chloro-2-pyridinyl)-4-méthylpipéridine obtenue dans l'étape (c) et de 80 ml d'acide chlorhydrique 6M. On concentre la solution en ajoutant du méthanol pour former un azéotrope et on cristallise le résidu dans de l'éthanol absolu.
On obtient ainsi 2,5 g du composé indiqué dans le titre (p.f. ≈300° C).

| Analyse élémentaire : | | |
|---|---|---|
| - trouvé | N basique = 5,38 ; | Cl tot = 27,2 |
| - calculé | N basique = 5,38 ; | Cl tot = 27,1 |

### EXEMPLE 2

### Maléate de 4-amino-1-(4-chloro-2-pyrimidinyl)-4-méthylpipéridine

**a) 1-benzyl-4-acétylamino-4-méthylpipéridine - formule (VIII), Alk = méthyle, R**^{**·**}**= acétyle, R=benzyle.**
Le produit est obtenu comme décrit dans l'exemple 1 étape (a).
**a')4-amino-1-benzyl-4-méthylpipéridine - formule (VIII), Alk = méthyle, R**^{**·**} **= H, R=benzyle.**
   On chauffe au reflux une solution de 26,35 g (0,113 mole) de 1-benzyl-4-acétylamino-4-méthylpipéridine, obtenue selon l'étape (a), dans 200 ml d'acide chlorhydrique 6N pendant 48 heures. On laisse refroidir et on ajoute une solution de NaOH à 30 % jusqu'à pH basique. On extrait au chlorure de méthylène, on sèche sur Na₂SO₄ et on évapore à sec. On purifie le résidu par chromatographie en éluant avec CH₂Cl₂/MeOH = 9/1. On obtient 16 g d'huile jaune correspondant au composé du titre (spectre IR/Film:2920 cm⁻¹).
**a'')1-benzyl-4-t-butoxycarbonylamino-4-méthylpipéridine - formule (VIII), Alk = méthyle, R**^{**·**} **= BOC, R = benzyle.**
   On dissout 15,5 g (0.081 mole) de 4-amino-1-benzyl-4-méthylpipéridine obtenue selon l'étape (a') dans 110 ml de chloroforme anhydre sous atmosphère d'azote et on y ajoute lentement 16,7 g (0,076 mole) de di-tert-butyl dicarbonate dissous dans 27 ml de chloroforme. On laisse agiter le mélange à la température ambiante pendant une nuit. On évapore le solvant et on obtient 24,0 g (0,079 mole) d'huile jaune correspondant au composé du titre (spectre IR: 3246 cm⁻¹, 1690 cm⁻¹).

**b) 4-t-butoxycarbonylamino-4-méthylpipéridine - formule (VIII), Alk = méthyle, R**^{**·**} **= BOC, R = H.**
On hydrogène un mélange de 24 g (0,079 mole) de 1-benzyl-4-t-butoxy carbonylamino-4-méthylpipéridine obtenue selon l'étape (a''), 1,5 g de Pd/C à 10 % et 250 ml d'alcool éthylique, à la pression ambiante. Après 24 heures environ, on filtre le catalyseur et on évapore à sec le solvant. On obtient ainsi 16,5 g du composé du titre (spectre IR/KBr: 3246 cm⁻¹, 1690cm⁻¹).
**c) 4-t-butoxycarbonylamino-1-(4-chloro-2-pyrimidinyl)-4-methylpipéridine - formule (VIII) Alk=méthyle, R**^{**·**} **= BOC, R = structure (IX) où Hal = Cl, X=Y=CH, Z=N et 4-t-butoxycarbonylamino-1-(2-chloro-4-pyrimidinyl)-4-méthylpipéridine - formule (VIII) Alk=méthyle, R**^{**·**} **= BOC, R = structure (IX) où Hal = Cl, Y=Z=CH, X=N.**
On chauffe au reflux pendant 24 heures un mélange de 3,13 g (0,0146 mole) de 4-t-butoxycarbonylamino-4-méthylpipéridine obtenue selon l'étape (b), 2,17 g (0,0146 mole) de 2,4-dichloropyrimidine, 1,48 g (0,0146 mole) de triéthylamine et 100 ml de toluène anhydre. On laisse refroidir, élimine par filtration les sous-produits de réaction et on évapore le solvant. Le résidu est constitué d'un mélange des deux isomères indiqués dans le titre et présente deux taches en T.L.C. Les deux composés sont séparés par chromatographie sur colonne de gel de silice en éluant avec CH₂Cl₂/MeOH = 98/2. On obtient ainsi deux produits:
- 1,2 mg de 4-t-butoxycarbonylamino-1-(4-chloro-2-pyrimidinyl)-4-méthylpipéridine sous forme d'huile jaune pâle, correspondant à la première tache éluée (spectre IR /KBr, 3245 cm⁻¹, 1675 cm⁻¹) et
- 3,3 g de 4-t-butoxycarbonylamino-1-(2-chloro-4-pyrimidinyl)-4-méthylpipéridine, p.f. 119-120° C, sous forme d'un solide blanc (correspondant à la deuxième tache éluée) .

**d) Maléate de 4-amino-1-(4-chloro-2-pyrimidinyl)-4-méthylpipéridine**
On refroidit une solution de 2,22 g (0,0068 mole) de 4-t-butoxycarbonylamino-1-(4-chloro-2-pyrimidinyl)-4-méthylpipéridine (étape c) dans 25 ml de chlorure de méthylène anhydre, sous atmosphère d'azote et on y ajoute lentement 5 ml d'anisole et 25 ml d'acide trifluoroacétique. On laisse agiter le mélange 1 heure à la température ambiante et on y ajoute lentement 25 ml d'une solution de NaOH à 30%. On extrait au chlorure de méthylène, on sèche sur Na₂SO₄ et on évapore à pression réduite le solvant et l'anisole. Le résidu est purifié par chromatographie en éluant avec CH₂Cl₂/MeOH = 9/1. On obtient ainsi la 4-amino-1-(4-chloro-2-pyrimidinyl)-4-méthylpipéridine base, qui est traitée avec de l'acide maléique dans un mélange éthanol/éther isopropylique. Le maléate ainsi obtenu est isolé par filtration (p.f. 154-155°C).

| Analyse pour C₁₀ H₁₅ ClN₄, C₄H₄O₄ : | | | |
|---|---|---|---|
| - calculé : | C 49,06 - | H 5,59 - | N 16,34 |
| - trouvé : | C 47,68 - | H 5,70 - | N 15,70 |

### EXEMPLE 3

### Maléate de 4-amino-1-(2-chloro-4-pyrimidinyl)-4-méthylpipéridine

On suit le mode opératoire décrit dans l'exemple 2, jusqu'à la fin de l'étape (c). On refroidit une solution de 6,81 g (0,021 mole) de 4-t-butoxycarbonylamino-1-(2-chloro-4-pyrimidinyl)-4-méthylpipéridine (étape c) dans 80 ml de chlorure de méthylène anhydre, sous atmosphère d'azote et on y ajoute lentement 16,4 ml d'anisole et 83 ml d'acide trifluoroacétique. On laisse agiter le mélange 1 heure à la température ambiante et on y ajoute lentement 80 ml d'une solution de NaOH à 30%. On extrait au chlorure de méthylène, on sèche sur Na₂ SO₄ et on évapore à pression réduite le solvant et l'anisole. On obtient ainsi la 4-amino-1-(2-chloro-4-pyrimidinyl)-4-méthylpipéridine base qui est traitée avec de l'acide maléique dans un mélange éthanol/éther éthylique. Le maléate ainsi obtenu est isolé par filtration (rendement 65%, p.f. 125-126°C).

| Analyse pour C₁₀ H₁₅ ClN₄, C₄H₄O₄ : | | | |
|---|---|---|---|
| - calculé : | C 49,06 - | H 5,59 - | N 16,34 |
| - trouvé : | C 48,05 - | H 5,70 - | N 15,76 |

### EXEMPLE 4

### 4-Acétylamino-1-(4-chloro-2-pyrimidinyl)-4-méthylpipéridine- formule (VIII) Alk=méthyle, R^{·} = acétyl, R = structure (IX) où Hal = Cl, X=Y=CH, Z=N et 4-acétylamino-1-(2-chloro-4-pyrimidinyl)-4-méthylpipéridine- formule (VIII) Alk=méthyle, R^{·} = acétyle, R = structure (IX) où Hal = Cl, Y=Z=CH, X=N.

**a) et b)** On suit le mode opérationnel décrit dans l'exemple 3 étapes (a) et (b).
**c)** On chauffe au reflux pendant 36 heures un mélange de 0,8 g (0,0051 mole) de 4-méthyl-4-acétylaminopipéridine obtenue selon l'étape (b), 0,76 g (0,0051 mole) de 2,6-dichloropyrimidine et 0,51 g (0,0051 mole) de triéthylamine dans 10 ml de toluène. On laisse refroidir, on filtre les sous-produits et on évapore le solvant à sec. On obtient 0,9 g d'huile jaune qui est constitué d'un mélange de deux isomères indiqués dans le titre et présente deux taches en T.L.C. Les deux composés sont séparés par chromatographie sur colonne de gel de silice en éluant avec CH₂Cl₂/MeOH = 95/5. On isole ainsi deux produits:
   - 120 mg de 4-acétylamino-1-(4-chloro-2-pyrimidinyl)-4-méthylpipéridine, p.f. 118- 120°C, correspondant à la première tache éluée et
   - 320 mg de 4-acétylamino-1-(2-chloro-4-pyrimidinyl)-4-méthylpipéridine, p.f. 173-175° C, correspondant à la deuxième tache éluée.

### EXEMPLE 5

### Maléate de 4-amino-1-(6-chloro-2-pyrazinyl)-4-méthylpipéridine

**a)** et **b)** On suit le mode opérationnel décrit dans l'exemple 1 étapes (a) et (b).
**c) 4-acétylamino-1-(6-chloro-2-pyrazinyl)-4-méthylpipéridine - formule (VIII), Alk=méthyle, R**^{**·**} **= acétyle, R = structure (IX) où Hal = Cl, X=Z=CH, Y=N.**
On chauffe au reflux pendant 24 heures un mélange de 1,0 g (0,0064 mole) de 4-acétylamino-4-méthylpipéridine, 0,95 g (0,0064 mole) de 2,6-dichloropyrazine, 0,65 g (0,0064 mole) de triéthylamine dans 10 ml de toluène. On laisse refroidir, on élimine par filtration les sous-produits de réaction et on évapore le solvant sous pression réduite. On purifie le résidu par chromatographie en éluant avec CH₂Cl₂/MeOH = 98/2, et on obtient une huile qui solidifie par traitement avec 10 ml d'ether éthylique (p.f. 147-148°C).
**d) Maléate de 4-amino-1-(6-chloro-2-pyrazinyl)-4-méthylpipéridine**
On chauffe au reflux pendant 14 heures 0,37 g (0,0013 mole) de 4-acétylamino-1-(6-chloro-2-pyrazinyl)-4-méthylpipéridine obtenue par l'étape (c) dans 10 ml d'acide chlorhydrique 6N. On laisse refroidir et on ajoute une solution de NaOH à 30 % jusqu'à pH alcalin. On extrait au chlorure de méthylène, on sèche sur Na₂SO₄ et on évapore à sec. On purifie le résidu par chromatographie en éluant avec CH₂Cl₂/MeOH = 7/3. On obtient ainsi la 4-amino-1-(6-chloro-2-pyrazinyl)-4-méthylpipéridine sous forme d'une huile qui est traitée avec de l'acide maléique dans un mélange éthanol/éther éthylique. Le maléate correspondant est isolé par filtration (p.f. 167-168°C).

| Analyse pour C₁₀ H₁₅ ClN₄, C₄H₄O₄ : | | | |
|---|---|---|---|
| - calculé : | C 49,05 - | H 5,59 - | N 16,35 |
| - trouvé : | C 48,67 - | H 5,42 - | N 15,93 |

### EXEMPLE 6

### Chlorhydrate de la 4-amino-1-(6-bromo-2-pyridinyl)-4-méthylpipéridine.

On suit le mode opérationnel de l'exemple 1 en utilisant la 2,6-dibromopyridine au lieu de la 2,6-dichloropyridine dans l'étape (d). On obtient ainsi la 4-amino-1-(6-bromo-2-pyridinyl)-4-méthylpipéridine sous forme de chlorhydrate.

## Revendications

1. Composé de formule (I) 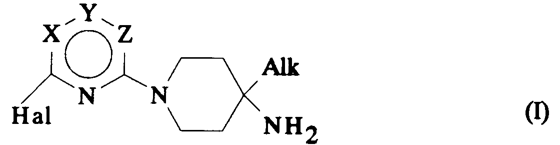 dans laquelle Hal représente un atome d'halogène, Alk représente un groupe (C₁-C₄) alkyle, X, Y et Z représentent chacun -CH= ou l'un d'entre eux est un atome d'azote et les autres représentent -CH=, ainsi que ses sels pharmaceutiquement acceptables ou non.

2. Composé de formule (I) selon la revendication 1, dans laquelle Alk est un groupe méthyle et Hal est un atome de chlore ou un de ses sels pharmaceutiquement acceptables ou non.

3. 4-amino-1-(6-chloro-2-pyridinyl)-4-méthylpipéridine et ses sels pharmaceutiquement acceptables.

4. Chlorhydrate de 4-amino-1-(6-chloro-2-pyridinyl)-4-méthylpipéridine.

5. 4-amino-1-(4-chloro-2-pyrimidinyl)-4-méthylpipéridine et ses sels pharmaceutiquement acceptables.

6. Maléate de 4-amino-1-(4-chloro-2-pyrimidinyl)-4-méthylpipéridine.

7. 4-amino-1-(2-chloro-4-pyrimidinyl)-4-méthylpipéridine et ses sels pharmaceutiquement acceptables.

8. Maléate de 4-amino-1-(2-chloro-4-pyrimidinyl)-4-méthylpipéridine.

9. 4-amino-1-(6-chloro-2-pyrazinyl)-4-méthylpipéridine et ses sels pharmaceutiquement acceptables.

10. Maléate ou chlorhydrate de 4-amino-1-(6-chloro-2-pyrazinyl)-4-méthylpipéridine.

11. Procédé pour la préparation des composés selon la revendication 1 caractérisé en ce que :
(a) on traite une 4-alkyl-1,2,3,6-tétrahydropyridine NH-protégée de formule (II) 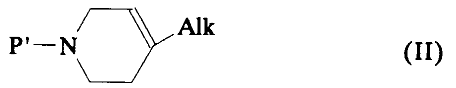 dans laquelle Alk représente un groupe alkyle de 1 à 4 atomes de carbone et P' est un groupe NH-protecteur clivable par réduction, avec acétonitrile et acide sulfurique et on isole une 4-acétylamino-4-alkylpipéridine NH-protégée de formule (III') 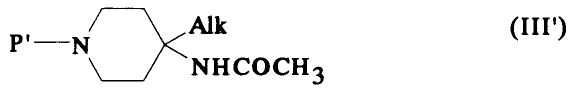 dans laquelle P' et Alk sont tels que définis ci-dessus, puis, éventuellement,
(a') on soumet le composé (III') ainsi obtenu à une NH₂-déprotection par hydrolyse pour obtenir une 4-alkyl-4-aminopipéridine NH-protégée de formule (IV) 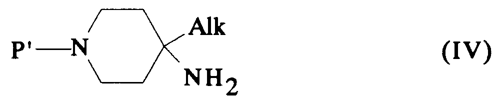 dans laquelle P' et Alk sont tels que définis ci-dessus; et
(a'') on protège le groupe amino primaire par un groupe, autre que le groupe acétyle, clivable par hydrolyse;
(b) on soumet la 4-alkyl-4-aminopipéridine diprotégée ainsi obtenue de formule (III) 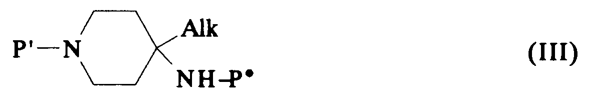 dans laquelle P' et Alk sont tels que définis ci-dessus et P^{·} représente un groupe NH₂-protecteur clivable par hydrolyse, à une NH-déprotection par réduction;
(c) on traite la 4-alkyl-4-aminopipéridine NH₂-protégée ainsi obtenue de formule (V) 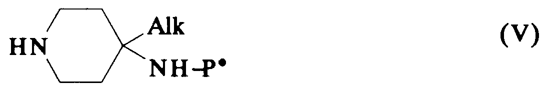 dans laquelle P^{·} et Alk sont tels que définis ci-dessus, avec un dihalohéteroaryle de formule (VI) 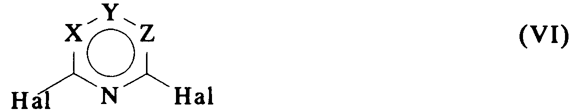 dans laquelle Hal est un atome d'halogène et X, Y et Z sont tels que définis ci-dessus;
(d) on hydrolyse le composé ainsi obtenu de formule (VII) 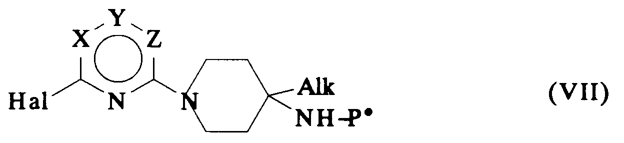 dans laquelle Hal, X, Y, Z, Alk et P^{·} sont tels que définis ci-dessus; et
(e) on isole le produit (I) ainsi obtenu tel quel ou sous forme d'un de ses sels ou on le transforme en un de ses sels.

12. Composé de formule (VIII) 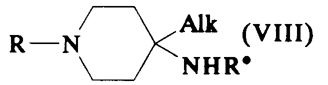 dans laquelle Alk représente un groupe (C₁-C₄) alkyle, R^{·} représente l'hydrogène ou un groupe protecteur clivable par hydrolyse, R représente l'hydrogène, un groupe protecteur clivable par réduction ou un hétérocycle de structure (IX) 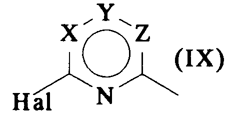 dans laquelle Hal représente un atome d'halogène, X, Y et Z représentent chacun -CH= ou l'un d'entre eux est un atome d'azote et les autres représentent -CH= ainsi que ses sels, étant donné que R^{·} est différent de l'hydrogène lorsque R représente l'hydrogène ou un groupe de structure (IX).

13. Utilisation d'un composé de formule (I) 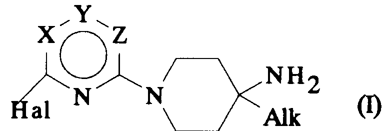 dans laquelle Hal représente un atome d'halogène, Alk représente un groupe (C₁-C₄)alkyle, X, Y et Z représentent chacun -CH= ou l'un d'entre eux est un atome d'azote et les autres représentent -CH=, ou d'un de ses sels pharmaceutiquements acceptables, pour la préparation d'un médicament destiné au traitement et/ou à la prophylaxie des troubles sérotoninergiques soit périphériques soit centraux, dans lesquels une action agoniste sélectivement médiée par les récepteurs 5HT₃ est demandée.

14. Utilisation selon la revendication 13 pour la préparation d'un médicament destiné au traitement et/ou à la prophylaxie des troubles dysthymiques, de la dépression, des troubles psychotiques, des cas d'anxiété ou des désordres de la motricité intestinale, tels que la constipation ou l'IBS.

15. Composition pharmaceutique contenant en tant que principe actif un composé de formule (I) 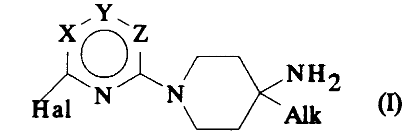 dans laquelle Hal représente un atome d'halogène, Alk représente un groupe (C₁-C₄)alkyle, X, Y et Z représentent chacun -CH= ou l'un d'entre eux est un atome d'azote et les autres représentent -CH=, ou un de ses sels pharmaceutiquements acceptables.

16. Composition pharmaceutique selon la revendication 15 contenant en tant que principe actif un composé de formule (I) où Hal représente un atome de chlore et Alk représente un groupe méthyle, ou un de ses sels pharmaceutiquement acceptables.

17. Composition pharmaceutique contenant, en tant que principe actif, un composé selon l'une quelconque des revendications de 3 à 10.
